# EUROPEAN PATENT SPECIFICATION

(11) **EP 3 250 051 B1**
(45) Date of publication and mention of the grant of the patent: **07.11.2018**
(21) Application number: 16702366.2
(22) Date of filing: 26.01.2016
(51) Int. Cl.: A23L 2/52, A23L 2/60, A23L 27/12

(54) **A NATURAL SWEETENING COMPOSITION OF LUO HAN GUO AND APPLE**
NATÜRLICHE SÜSSSTOFFZUSAMMENSETZUNG AUS LUO HAN GUO UND APFEL
COMPOSITION ÉDULCORANTE NATURELLE À BASE DE LUO HAN GUO ET DE POMME

(30) Priority: 27.01.2015 TR 201500903; 06.03.2015 TR 201502736
(43) Date of publication of application: 06.12.2017
(73) Proprietor: Montero Gida Sanayi Ve Ticaret A.S., 34460 Istanbul (TR)
(72) Inventor: TOKSÖZ, Ahmet, 34460 Istanbul (TR); TOKSÖZ, Zafer, 34460 Istanbul (TR); MUTLU, Onur, 34460 Istanbul (TR); TÜRKYILMAZ, Ali, 34460 Istanbul (TR); YELKEN, Gülay, 34460 Istanbul (TR); ZAN, Merve, 34460 Istanbul (TR)
(74) Representative: Sevinç, Erkan
(86) International application number: PCT/EP2016/051493
(87) International publication number: WO 2016/120228

(56) References cited:
- WO-A1-2008/112866
- US-A1- 2006 003 053
- US-A1- 2009 162 529
- DATABASE WPI Week 200440 Thomson Scientific, London, GB; AN 2004-427578 XP002755012, & KR 2004 0014623 A (HAITAI DAIRY CO LTD) 14 February 2004 (2004-02-14)

## Description

### Field of Invention

The present invention relates to a natural sweetening composition comprising apple concentrate having a median particle size (d50) greater than 100 µm and Luo Han Guo concentrate having a median particle size (d50) greater than 1 µm and use in beverage, food, pharmaceutical, oral, dietetic, veterinary or tobacco products thereof.

### Background of Invention

Luo Han Guo (luohanguo) refers to the fruit of Siraitia grosvenori, formerly called Momordica grosvenori, a member of the Curcubitaceae. The plant is cultivated for its fruit Luo Han Guo, whose extract is nearly 300 times sweeter than sugar and has been used in China as a natural low-calorie sweetener and in traditional Chinese medicine to treat diabetes and obesity. Luo Han Guo is popularly considered a longevity aid and is used to balance heat buildup caused by internal conditions, life-forces, or external heat. It is used as an expectorant and antitussive to treat lung congestion, cough, other respiratory ailments, and sore throat. It also is used for constipation and chronic enteritis. Luo Han Guo is a low-caloric, low-glycemic food used as a natural sweetener in beverages and food.

Luo Han Guo is collected as a round green fruit that turns brown upon drying. The sweet taste of Luo Han Guo comes primarily from mogrosides, a group of terpene glycosides, present at the level of about 1% of the fleshy part of the fruit. Both the fresh and dried fruits are extracted to yield a powder that is 80% or more mogrosides. The mogrosides have been numbered, 1-5, and the main component is called mogroside-5, previously known as esgoside. Other, similar compounds from Luo Han Guo have been labeled siamenoside and neomogroside. The mixed mogrosides are estimated to be about 300 times as sweet as sugar by weight, so that the 80% extracts are nearly 250 times sweeter than sugar; pure mogrosides 4 and 5 may be 400 times as sweet as sugar by weight.

A process for making a useful sweetener from Luo Han Guo was patented in 1995 by Procter and Gamble Company in U.S. patent 5,411,755. As described in the patent application, the fruit itself, though sweet, has too many additional flavors that would make it unsuitable for widespread use as a sweetener, so P&G developed a method for processing it to eliminate the undesired flavors. In the P&G process, the fresh fruit is picked before ripening and allowed to complete its ripening during storage so that processing begins with the just-ripe fruit. The peel and seeds are then removed, and the mashed fruit becomes the basis of a concentrated fruit juice or puree that can be used in food manufacturing. Further processing involves using solvents to remove volatile and off-flavor components. Numerous sugar substitutes derived from Luo Han Guo by similar processes that isolate the sweet compounds are now readily available for manufacturing and for kitchen use.

Recent work on Luo Han Guo includes investigation of the antioxidant activity of the mogrosides (Shi H, et al., Antioxidant property of fructus momordicae extract, 1996 Biochemistry and Molecular Biology International 1996; 40 (6): 1111-1121.) and their potential use as cancer prevention compounds (Konoshima T and Takasaki M, Cancer-chemopreventive effects of natural sweeteners and related compounds, Pure Applied Chemistry 2002; 74(7): 1309-1316.). This suggested effect is based on the understanding that antioxidants can produce significant reversal or suppression of the early stage of cancer development, which has been an area of particular interest for tea drinking (Katiyar SK and Mukhtar H, Tea antioxidants in cancer chemoprevention, Journal of Cellular Biochemistry, Supplement 1997; 27: 59-67.). Further, Luo Han Guo and its sweetening component are often mentioned in relation to diabetes and obesity, because it can substitute for caloric sugars normally consumed in the diet.

Apple concentrate is a flavouring, colouring and sweetening extract that contains the fruits own natural sugars. It also contains the fruit acids, minerals and secondary plant substances of apples - the polyphenolics which are nowadays regarded as positive health promoters. These polyphenolic substances are attributed with anti-oxidative and cancer-protective properties and are also regarded to have a positive effect on blood glucose and hypertension as well as lowering the cholesterol level. Further it is presumed that the positive nutritional and physiological effects of apple minerals and vitamins are associated with these secondary plant substances. Apple concentrate is a natural alternative to one of our most popular but problematic food commodities, the sucrose. For both food WO 2008112866 A1 relates to beverages and other beverage products, such as beverage concentrates having formulations suitable to meet market demand for natural ingredients and alternative nutritional characteristics or flavor profiles in beverages. D1 discloses a list of natural sweeteners suitable for at least certain exemplary embodiments of the invention including apple concentrate and Luo Han Guo.

KR 20040014623 A relates to a method of mixing a variety of sugars (liquid fructose, oligosaccharides, aspartame, sucralose) with apple juice in a brown rice vinegar, mixing the apple fruit, plum flavor for producing good fresh brown rice vinegar drink.

US 2009162529 A1 provides a natural sweetener composition comprising powdered extracts of Luo Han Guo whole fruits, mogrosides, and one or more dietary fiber. In one embodiment, extracts obtained from Luo Han Guo whole fruits are first made into powder form. Mogrosides and/or mogroside-V are then added to the powder extract, which is further formulated with dietary fiber such as inulin into a natural sweetener composition.

US 2006003053 A1 discloses methods for extracting juice from plant material containing terpene glycosides and the use of this juice in food, beverage and health care compositions. Terpene glycosides exist in a variety of plant and botanical sources. One excellent source of terpene glycosides is certain members of the Cucurbitaceae family, and in particular, Luo Han Guo fruit, otherwise known as Siraitia grosvenorii. producers and consumers they offer various advantages to traditional sugar. Natural apple concentrate made from de-juiced and carefully dried apples that reflect the characteristic sugar spectrum, balancing the nutritional and physiological profiles with the consumers association of apple as symbol for health and vitality. The apple concentrate does not contain sucrose but a very high content of fructose.

Due to these neutral properties the potential application for natural sweetener composition is diverse, so this high quality sweetener can be used to replace sucrose in virtually every single food. Although sucrose, provides the most desirable taste to consumers, it is caloric and unhealthy. Furthermore, the natural sweetener composition is process friendly with virtually the same sweetening power as traditional sugar. But to be able to claim that you are using "natural sweetener" instead of sucrose provides a consumer friendly image and a positive sales argument. The carbohydrate fructose found in the natural sweetener composition is generally recognised as diabetic sweeteners, as compared to sucrose they are sugar substitutes and metabolised independent of insulin.

It has been also known numerous natural sweeteners are non-caloric; however, they exhibit sweet tastes that have different temporal profiles, maximal responses, flavor profiles, mouthfeels, and/or adaptation behaviors than that of sugar. Because of these differences, in a food or beverage, causes an unbalanced temporal profile and/or flavor profile. In addition to the difference in temporal profile, natural sweeteners generally exhibit:
(i) lower maximal response than sugar,
(ii) tastes including bitter, metallic, cooling, astringent, licorice-like taste, etc.,
(iii) sweetness which diminishes on iterative tasting.

It is well known to those skilled in the art of food/beverage/pharmaceutical composition that the sweetener in a composition requires balancing of the flavor and other taste components. If the taste profile of natural sweeteners could be modified to impart specific desired taste characteristics to be more sugar-like, the type and variety of compositions that may be prepared with that sweetener would be expanded significantly. Accordingly, it would be desirable to selectively modify the taste characteristics of natural sweeteners.

Another important point for the food/beverage/pharmaceutical composition is processability. The natural sweetening composition must be easily handled, mixed with excipients even having a larger particle size and must be processed into beverage, food, pharmaceutical, oral, dietetic, veterinary or tobacco products without the known and expected problems such as segregation or de-mix during the process steps. It is usually related with the particle size and particle size distribution. For the natural sweetener compositions, it is not always easy to achieve content uniformity, aggregation and large particle size can be occurred during the process.

In this invention it is surprisingly found that, spesific particle size of each apple and Luo Han Guo concentrates also gives homogeneity and high flowability properties to the composition during the process. And thus change the taste balance of a food or beverage or pharmaceutical compositions. They are slower in onset and longer in duration than the sweet taste produced by sucrose.

### Description of the invention

Health trends have promoted an increased use of natural sweeteners in consumer diets. Amongst the many possibilities available to product developers is the option to enrich foods with "healthy ingredients" or perhaps to exchange one or more ingredients of the recipe in order to optimise the nutritional profile.

For instance foods, which do not include additives or sugar, are perceived by the consumer, as natural and healthy. Despite their immense popularity, sweeteners and particularly sucrose recently gained bad publicity because of health concerns. That's why fruit sweeteners are becoming more and more important as a natural alternative to sucrose as they contain the fruit's own sugar spectrum.
Of increasing nutritional and physiological importance to today's consumer is the glycemic index (GI) that is much lower for fructose compared to that of glucose. Using glucose as the standard base, with a GI of 100, fructose has only a GI of around 20. The GI measures the glycemic response (an indication of the rate at which the blood glucose level rises and how it is sustained over time) after ingestion of carbohydrate foods. The consumption of food with low GI, such as fruit and vegetables, results in a slow increase of the blood glucose level. Food with a high GI, such as sucrose and white bread, results in a quick response of blood glucose insulin levels and therefore should not only be avoided by diabetics, but also by the growing number of health conscious consumers as there is growing evidence that over long time, a diet based on high-carbohydrate low-glycemic foods is beneficial towards health by preventing chronic diseases such as diabetes, coronary heart disease and possibly cancer.

In present invention, the phrase "concentrate" includes any apple or Luo Han Guo product that is solid comprising "extract", "concetrated extract", "dried fruit", "de-juiced fruit", "lyophilized", "spray dried", "crystallized" or mixtures thereof.

In present invention, the concentrates comprising fructose, aromas, flavorings, vitamins, minerals, nutrients, concentrated juices, pieces of pulp and other remnants or mixtures thereof.

The main object of the present invention is to obtain adequate content uniformity and desired particle size distribution of natural sweetening composition comprising apple concentrate and Luo Han Guo concentrate and improved processes. In this invention, the natural sweetening composition has homogeneity and high flowability properties during the process.

Another main object of this invention is to obtain a sweetener composition that has sweeter taste because of the synergy between apple concentrate and Luo Han Guo concentrate. This invention relates to a natural sweetening composition comprising apple concentrate and Luo Han Guo concentrate, the composition is exhibiting synergy, i. e. providing greater sweetness than would be expected from simple summation of the sweetness contributed by the component sweetening agents.

Another main object of the present invention is to obtain a sweetener composition that does not increase the glycemic index. This invention provides a sweetener composition, which does not raise the glycemic index and provides the advantage of production simplicity.

Another main object of the present invention is to obtain a sweetener composition that is natural and healthy. Also it also would be desirable to improve the taste of ingestible compositions that include natural ingredients to promote their use and the resulting health benefits. In this invention, the natural sweeteners apple concentrate and Luo Han Guo concentrate do not carry any glycemic index potential. Thus, a product is obtained which can be used by those who are in diets or by the diabetics.

The present invention provides a novel natural sweetening composition comprising apple concentrate having a median particle size (d50) greater than 100 µm and Luo Han Guo concentrate having a median particle size (d50) greater than 1 µm which overcomes the above described problems in prior art and have additive advantages over them.

In an embodiment, the natural sweetening composition comprising; apple concentrate having a median particle size (d50) is between 100 and 500 µm and Luo Han Guo concentrate having a median particle size (d50) is between 1 and 50 µm.

In an embodiment, the natural sweetening composition of this invention comprises apple concentrate having a median particle size (d50) greater than 100 µm, preferably greater than 250 µm, more preferably greater than 300 µm and having particle size of d(90) greater than 600 µm, preferably greater than 750 µm.

In a more preferably embodiment, the natural sweetening composition of this invention comprises apple concentrate having a median particle size (d50) greater than 300 µm and having particle size of (d90) greater than 750 µm.

In another embodiment, the natural sweetening composition of this invention comprises Luo Han Guo concentrate having a median particle size (d50) greater than 1 µm, preferably greater than 5 µm, more preferably greater than 10 µm and having particle size of (d90) greater than 20 µm, preferably greater than 30 µm.

In a more preferably embodiment, the natural sweetening composition of this invention comprises Luo Han Guo concentrate having a median particle size (d50) greater than 10 µm and having particle size of (d90) greater than 30 µm.

As used here in, "particle size distribution" is defined by the cumulative volume size distrubition as tested by a conventionally accepted method which is the laser diffraction method determined by the equipment of Malvern Mastersizer 2000. "Median particle size" is defined by "d50", the diameter where fifty percent of the distribution has a smaller particle size and "d90" means that the the diameter where ninety percent of the distribution has a smaller particle size. "Desired particle size distribution" is defined by the ratio between the median particle size (d50) and the particle size at (d90).

In order to achieve the unexpected good results of content uniformity the natural sweetening composition of this invention the apple concentrate and Luo Han Guo concentrate must have a desired particle size distribution.

The natural sweetening composition of the present invention comprising; apple concentrate having a particle size ratio of (d50) to (d90) which is between 0.30 and 0.80; Luo Han Guo concentrate having a particle size ratio of (d50) to (d90) which is between 0.20 and 0.70.

According to this embodiment, this ratio of (d50) to (d90) of apple concentrate must be between 0.30 and 0.80, preferably between 0.35 and 0.70, more preferably between 0.40 and 0.60, and most preferably between 0.40 and 0.50.

According to this embodiment, this ratio of (d50) to (d90) of Luo Han Guo concentrate must be between 0.20 and 0.70, preferably between 0.25 and 0.60, more preferably between 0.30 and 0.50, and most preferably between 0.30 and 0.40.

The natural sweetening composition composition of the present invention, comprising apple concentrate and Luo Han Guo concentrate wherein the particle size ratio of d(50) of Luo Han Guo concentrate to apple concentrate is between 0.01 to 0.1.

According to this embodiment, particle size ratio of d(50) of Luo Han Guo concentrate to apple concentrate is between 0.01 to 0.1, preferably between 0.02 and 0.08, more preferably between 0.03 and 0.07 and and most preferably between 0.03 and 0.06.

According to the special particle sizes and ratios mentioned below, the invention's advantages are resonable preparing process, high flowability, uniform dispersion of sweetening agent and low moisture absorption. Specifically, this invention of the natural sweetening composition has the advantage of minimal changes in the products' physical characteristics, such as density, flowability, etc.; namely, preventing the final composition from becoming sticky which often occurs when a sweetening agent is added into a composition rather, and retains more of the characteristic flowability and density of the bulked composition with is normally adversely affected by conventional sweetening agent.

In this present invention, the natural sweetening composition further comprising inuline, erythritol, coconut sugar, lactose, locust bean gum; rebaudioside A, rebaudioside B, rebaudioside C, rebaudioside D, rebaudioside E, rebaudioside F, dulcoside A, dulcoside B, rubusoside, isomogroside V, 11-oxomogroside, steviol glycosides,stevia, stevioside, mogroside IV, mogroside V, mogroside VI, siamenoside, monatin and its salts (monatin SS, RR, RS, SR), curculin, glycyrrhizin , glycyrrhizic acid and its salts, thaumatin, monellin, mabinlin, brazzein, hernandulein, phyllodulcin, glycyphyllin, phloridzim, trilobatin, baiyunoside, osladin, polypodoside A, pterocaryoside A, pterocaryoside B, mukurozioside, phlomisoside I, periandrin I, abrusoside A, cyclocarioside I; a sweetener which is extracted from the group comprising Stevia rebaudiana, Glycyrrhiza glabra, Siraitia grosvenorii; sucrose, liquid sucrose, glucose, liquid glucose, fructose, liquid fructose, dextrose, galactose, lactulose, lactose, cellobiose, kojibiose, nigerose, isomaltose, .beta.,.beta.- trehalose, .alpha.,.beta.-trehalose, tagatose, sophorose, laminaribiose, gentiobiose, turanose, maltose, maltulose, palatinose, gentiobiulose, mannobiose, melibiose, melibiulose, rhamnose, ribose, rutinose, rutinulose, trehalose,xylobiose, xylose, corn syrups, fructo-oligosaccharides; erythritol, glycol, glycerol, threitol, arabitol, xylitol, ribitol, mannitol, sorbitol, dulcitol, iditol, isomalt, maltitol, lactitol, polyglycitol; apple skin extract (applephenon) or mixtures thereof.

In one embodiment, the natural sweetening composition comprising apple concentrate and Luo Han Guo concentrate, inuline.

In another embodiment, the natural sweetening composition comprising apple concentrate and Luo Han Guo concentrate, erythritol.

In another embodiment, the natural sweetening composition comprising apple concentrate and Luo Han Guo concentrate, coconut sugar.

In another embodiment, the natural sweetening composition comprising apple concentrate and Luo Han Guo concentrate, lactose.

In another embodiment, the natural sweetening composition comprising apple concentrate and Luo Han Guo concentrate, locust bean gum.

In one embodiment, the natural sweetening composition comprising apple concentrate and Luo Han Guo concentrate, inuline, erythritol.

In one embodiment, the natural sweetening composition comprising apple concentrate and Luo Han Guo concentrate, inuline, erythritol, coconut sugar.

In one embodiment, the natural sweetening composition comprising apple concentrate and Luo Han Guo concentrate, inuline, erythritol, locust bean gum.

In one embodiment, the natural sweetening composition comprising apple concentrate and Luo Han Guo concentrate, inuline, erythritol, coconut sugar, lactose.

In one embodiment, the natural sweetening composition comprising apple concentrate and Luo Han Guo concentrate, inuline, erythritol, coconut sugar, lactose, locust bean gum.

Use of the natural sweetening composition comprising apple concentrate and Luo Han Guo concentrate in beverage, food, pharmaceutical, oral, dietetic, veterinary or tobacco products

In this present invention, said products are solid, liquid or semisolid.

Use of the natural sweetening composition, wherein;
- said beverages are selected from the group comprising colas, ginger ales, root beers, ciders, fruit-flavored soft drinks, powdered soft drinks or mixtures thereof; fruit juices originating in fruits or vegetables, fruit juices including squeezed juices or mixtures thereof, fruit juices containing fruit particles, fruit beverages, fruit juice beverages, beverages containing fruit juices, beverages with fruit flavorings, vegetable juices, juices containing vegetables, and mixed juices containing fruits and vegetables; drinks, sport drinks, energy drinks, near water or mixtures thereof; tea type or favorite type beverages which are coffee, cocoa, chocolate, black tea, green tea, oolong tea or mixtures thereof; beverages containing milk components which are milk beverages, coffee containing milk components, cafe au lait, milk tea, fruit milk beverages, drinkable yogurt, lactic acid bacteria beverages or mixtures thereof; dairy products; alcoholic beverages which are wine, beer, cider; distilled beverages which are spirit, liquor;
- said food products are selected from the group comprising bakery products; desserts which are yogurt, jellies, drinkable jellies, puddings, Bavarian cream, blancmange, cakes, brownies, mousse or mixtures thereof; sweetened food products eaten at tea time or following meals; frozen foods; cold confections, types of ice cream which are ice cream, ice milk, lacto-ice or mixtures thereof; ice confections which are sherbets, dessert ices or mixtures thereof; ice cream; general confections, baked confections or steamed confections which are cakes, crackers, biscuits, buns with bean-jam filling or mixtures thereof; rice cakes and snacks; nut and peanut butter; table top products; general sugar confections which are chewing gum, hard candy, soft candy, mints, nougat candy, jelly beans or mixtures thereof; chocolates, chocolate creams; sauces including fruit flavored sauces, chocolate sauces or mixtures thereof; edible gels; cremes including butter cremes, flour pastes, whipped cream or mixtures thereof; jams including strawberry jam, marmalade or mixtures thereof; breads including sweet breads or other starch products or mixtures thereof; spice; general condiments including seasoned soy sauce used on roasted meats, roast fowl, barbecued meat or mixtures thereof, as well as tomato catsup, sauces, noodle broth or mixtures thereof; processed agricultural products, livestock products or seafood; processed meat products which are sausage or mixtures thereof; retort food products, pickles, preserves boiled in soy sauce, delicacies, side dishes; snacks which are potato chips, cookies or mixtures thereof; wafer, waffle, cornets, bars, wafer sheet; cereal products, granola; baby food;
- said pharmaceutical products are selected from the group comprising drugs or quasi-drugs that are administered orally or used in the oral cavity, wherein the drug may be in solid, liquid, gel or gas form which is a pill, tablet, spray, capsule, syrup, drop, troche agent, sachet, powder or mixtures thereof; herbal products, vitamins; nutraceutical products comprising any food or part of a food that may provide medicinal or health benefits, including the prevention and treatment of disease;
- said oral products are selected from the group comprising hygienic or cosmetic products; personal care products which are oral compositions used in the oral cavity, mouth freshening agents, gargling agents, mouth rinsing agents, toothpaste, tooth polish, dentrifices, mouth sprays, teeth-whitening agents or mixtures thereof;
- said dietetic products are selected from the group comprising dietary supplement;
- said veterinary products are selected from the group comprising animal feed;
- said tobacco products are selected from the group comprising smoke and smokeless tobacco products such as snuff, cigarette, pipe and cigar tobacco, and all forms of tobacco such as shredded filler, leaf, stem, stalk, homogenized leaf cured, reconstituted binders and reconstituted tobacco from tobacco dust, fines or ether sources in sheet, pellet or other forms, tobacco substitutes formulated from non-tobacco materials, dip or chewing tobacco.

In result, the art of sweetener products requires a novelty to provide the advantages of being healthy and natural, not increasing the glycemic index, production simplicity, cost-efficient and sweeter taste because of the synergy between apple concentrate and Luo Han Guo concentrate. It has been found that contrary to prior art and the general knowledge of the skilled person in the natural sweetening composition of comprising apple concentrate having a median particle size (d50) greater than 250 µm and Luo Han Guo concentrate having a median particle size (d50) greater than 5 µm does not show the expected disadvantages in flow behaviour and homogeneity and thus processability if it is provided as particles having a desired particle size distribution. In this case the sweetening composition can be easily handled, mixed with excipients even having a larger particle size and can be processed into beverage, food, pharmaceutical, oral, dietetic, veterinary or tobacco products without the known and expected problems such as segregation or de-mix during the process steps.

According to this invention, some examples are disclosed below:

### Example 1: Chocolate formulation

| **Ingredients** | **Amount (% by weight)** |
|---|---|
| Erythrol | 5 - 25 |
| Inulin | 20 - 65 |
| Natural sweetening composition of apple and LHG concentrates | 1 - 35 |
| Hazelnut or puree thereof | 10 - 30 |
| Cacao products | 1 - 45 |
| Milk products | 5 - 30 |
| Vegetable oil | 25 - 40 |
| Lactose | 0 - 5 |
| Vanilla | 0 - 2 |

Production process of the formulation as following:
The mixture of erythrol, natural sweetening composition of apple and LHG concentrates, and inulin is finely mixed and homogenized by means of a mixer. Then, a previously-grinded hazelnut puree is added to this mixture. The resulting mixture is grinded and mixed back so as to be homogenized.

To the preferably pre-ground mixture is added a liquid mixture composed of cacao powder, dry milk products, lactose and vanilla if required, and vegetable oil. Then, conching and settling steps are applied to this mixture, where after the resultant mixture is prepared for sales, following a filling or shaping process as required by the form of the targeted product. The process steps may be illustrated in the following diagram.

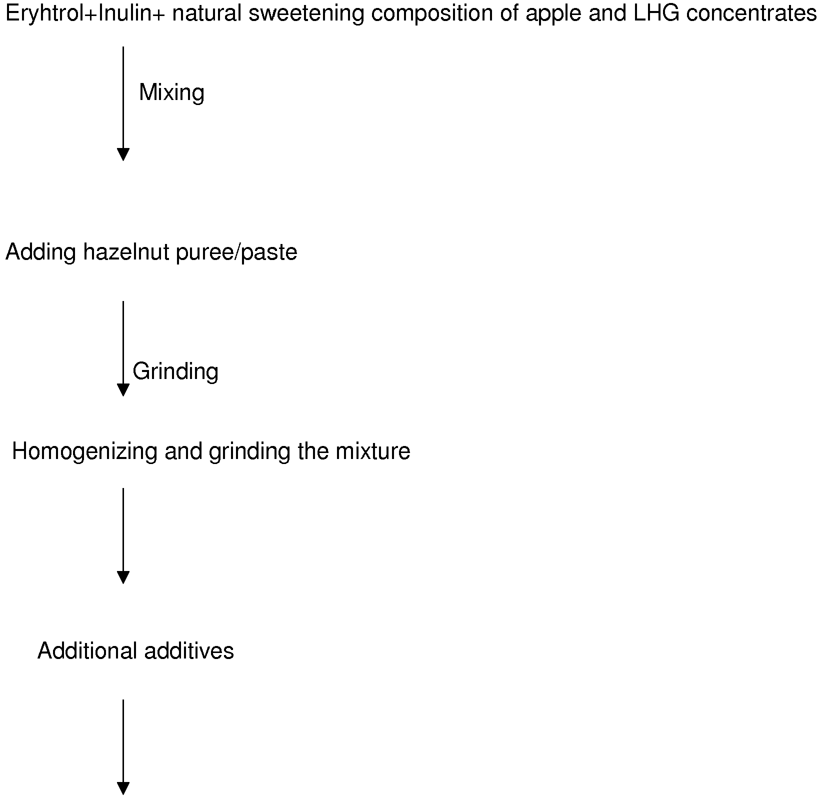

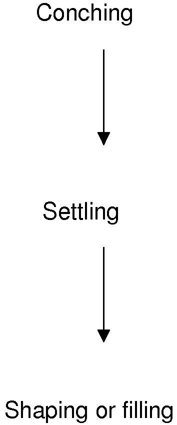

In result, thanks to the embodiment disclosed above, dietary chocolate composition comprising natural sweetening composition of apple and LHG concentrates is obtained, with reduced calorie and low glycemic index.

### Example 2: Sweetener tablet formulation

| **Ingredients** | **Amount (% by weight)** | **Function** |
|---|---|---|
| Natural sweetening composition of apple and LHG concentrates | 5 - 95 | Sweetener |
| D-Mannitol | 50 - 80 | Filler |
| Croscarmellose sodium | 1 - 5 | Disintegrant |
| Colloidal silicone dioxide | 0.1 - 5 | Glidant |
| L-Leucine | 1 - 10 | Lubricant |
| Sodium stearyl fumarate | 1 - 5 | Lubricant |

Production process of the formulation as following:
Mannitol, L-leucine and a suitable disintegrant are added to natural sweetening composition of apple and LHG concentrates and mixed. A mixture of colloidal silicone dioxide and sodium stearyl fumarate is added to this first mixture and mixed them together. The resulting mixture is compressed in the form of tablets. The production preferably involves direct compressing method. It can alternatively be produced by wet granulation.

In this formulation, the sweetener is anatural sweetening composition of apple and LHG concentrates, the mannitol used as a filler, and other excipients do not carry any glycemic index potential. Thus, a product is obtained which can be used by those who are in diets or by the diabetics.

Sodium stearyl fumarate is a very efficient lubricant, being less hydrophobic and having a lower delaying effect, when the tablet is dissolved. The present invention involves the use of sodium stearyl fumarate and L-leucine (the lubricant) and colloidal silicone dioxide (the glidant) in the form of a combination, thereby eliminating the problems related to decreasing flowability and to the sticking of these materials to the production apparatuses and elements. Additionally, the product's dissolution rate is brought to a very satisfactory extent, thanks to using croscarmellose sodium as a disintegrant.

### Example 3: Oral pharmaceutical formulation

| **Ingredients** | **Amount (% by weight)** | **Function** |
|---|---|---|
| Active ingredient | 5 - 95 | Active ingredient |
| Anhydrous citric acid | 0.1 - 2 | Flavor enhancer |
| Hypromellose | 0.25 - 5 | Suspending and/or thickening agent |
| Natural sweetening composition of apple and LHG concentrates | 5 - 80 | Sweetener |
| Orange aroma | 0.5 - 2 | Aromatic agent |
| Sodium benzoate | 0.01 - 0.03 | Microbial preservative |
| Disodium EDTA | 0.005 - 0.1 | Antioxidant |

Production process of the formulation as following:
Active ingredient, hypromellose and anhydrous citric acid are mixed. Natural sweetening composition of apple and LHG concentrates, orange aroma, sodium benzoate and disodium EDTA are added to the first mixture and mixed and homogenized. The resulting mixture is filled into sachets.

## Claims

1. A natural sweetening composition comprising;
**a)** Apple concentrate having a median particle size (d50) greater than 100 µm,
**b)** Luo Han Guo concentrate having a median particle size (d50) greater than 1 µm.

2. The natural sweetening composition of claim 1, comprising;
**a)** Apple concentrate having a median particle size (d50) is between 100 and 500 µm,
**b)** Luo Han Guo concentrate having a median particle size (d50) is between 1 and 50 µm.

3. The natural sweetening composition of claim 1, comprising;
**a)** Apple concentrate having a particle size ratio of (d50) to (d90) which is between 0.30 and 0.80,
**b)** Luo Han Guo concentrate having a particle size ratio of (d50) to (d90) which is between 0.20 and 0.70.

4. The natural sweetening composition of claim 1, comprising apple concentrate and Luo Han Guo concentrate wherein the particle size ratio of d(50) of Luo Han Guo concentrate to apple concentrate is between 0.01 to 0.1.

5. The natural sweetening composition of claim 1, further comprising inuline, erythritol, coconut sugar, lactose, locust bean gum; rebaudioside A, rebaudioside B, rebaudioside C, rebaudioside D, rebaudioside E, rebaudioside F, dulcoside A, dulcoside B, rubusoside, isomogroside V, 11-oxomogroside, steviol glycosides,stevia, stevioside, mogroside IV, mogroside V, mogroside VI, siamenoside, monatin and its salts (monatin SS, RR, RS, SR), curculin, glycyrrhizin , glycyrrhizic acid and its salts, thaumatin, monellin, mabinlin, brazzein, hernandulein, phyllodulcin, glycyphyllin, phloridzim, trilobatin, baiyunoside, osladin, polypodoside A, pterocaryoside A, pterocaryoside B, mukurozioside, phlomisoside I, periandrin I, abrusoside A, cyclocarioside I; a sweetener which is extracted from the group comprising Stevia rebaudiana, Glycyrrhiza glabra, Siraitia grosvenorii; sucrose, liquid sucrose, glucose, liquid glucose, fructose, liquid fructose, dextrose, galactose, lactulose, lactose, cellobiose, kojibiose, nigerose, isomaltose, .beta.,.beta.- trehalose, .alpha.,.beta.-trehalose, tagatose, sophorose, laminaribiose, gentiobiose, turanose, maltose, maltulose, palatinose, gentiobiulose, mannobiose, melibiose, melibiulose, rhamnose, ribose, rutinose, rutinulose, trehalose,xylobiose, xylose, corn syrups, fructo-oligosaccharides; erythritol, glycol, glycerol, threitol, arabitol, xylitol, ribitol, mannitol, sorbitol, dulcitol, iditol, isomalt, maltitol, lactitol, polyglycitol; apple skin extract (applephenon) or mixtures thereof.

6. The natural sweetening composition of claim 5, wherein the composition comprising apple concentrate, Luo Han Guo concentrate and inuline.

7. The natural sweetening composition of claim 5, wherein the composition comprising apple concentrate, Luo Han Guo concentrate and erythritol.

8. The natural sweetening composition of claim 5, wherein the composition comprising apple concentrate, Luo Han Guo concentrate and coconut sugar.

9. The natural sweetening composition of claim 5, wherein the composition comprising apple concentrate, Luo Han Guo concentrate and lactose.

10. The natural sweetening composition of claim 5, wherein the composition comprising apple concentrate, Luo Han Guo concentrate and locust bean gum.

11. The natural sweetening composition of claim 5, wherein the composition comprising apple concentrate, Luo Han Guo concentrate, inuline and erythritol.

12. The natural sweetening composition of claim 5, wherein the composition comprising apple concentrate, Luo Han Guo concentrate, inuline, erythritol and coconut sugar.

13. The natural sweetening composition of claim 5, wherein the composition comprising apple concentrate, Luo Han Guo concentrate, inuline, erythritol and locust bean gum.

14. The natural sweetening composition of claim 5, wherein the composition comprising apple concentrate, Luo Han Guo concentrate, inuline, erythritol, coconut sugar and lactose.

15. The natural sweetening composition of claim 5, wherein the composition comprising apple concentrate, Luo Han Guo concentrate, inuline, erythritol, coconut sugar, lactose and locust bean gum.

16. Use of the natural sweetening composition comprising apple concentrate and Luo Han Guo concentrate as in any one of claims 1-15 in beverage, food, pharmaceutical, oral, dietetic or veterinary products.

17. Use of the natural sweetening composition of claim 16, wherein said products are solid, liquid or semisolid.

18. Use of the natural sweetening composition of claim 17, wherein;
- said beverages are selected from the group comprising colas, ginger ales, root beers, ciders, fruit-flavored soft drinks, powdered soft drinks or mixtures thereof; fruit juices originating in fruits or vegetables, fruit juices including squeezed juices or mixtures thereof, fruit juices containing fruit particles, fruit beverages, fruit juice beverages, beverages containing fruit juices, beverages with fruit flavorings, vegetable juices, juices containing vegetables, and mixed juices containing fruits and vegetables; drinks, sport drinks, energy drinks, near water or mixtures thereof; tea type or favorite type beverages which are coffee, cocoa, chocolate, black tea, green tea, oolong tea or mixtures thereof; beverages containing milk components which are milk beverages, coffee containing milk components, cafe au lait, milk tea, fruit milk beverages, drinkable yogurt, lactic acid bacteria beverages or mixtures thereof; dairy products; alcoholic beverages which are wine, beer, cider; distilled beverages which are spirit, liquor;
- said food products are selected from the group comprising bakery products; desserts which are yogurt, jellies, drinkable jellies, puddings, Bavarian cream, blancmange, cakes, brownies, mousse or mixtures thereof; sweetened food products eaten at tea time or following meals; frozen foods; cold confections, types of ice cream which are ice cream, ice milk, lacto-ice or mixtures thereof; ice confections which are sherbets, dessert ices or mixtures thereof; ice cream; general confections, baked confections or steamed confections which are cakes, crackers, biscuits, buns with bean-jam filling or mixtures thereof; rice cakes and snacks; nut and peanut butter; table top products; general sugar confections which are chewing gum, hard candy, soft candy, mints,
nougat candy, jelly beans or mixtures thereof; chocolates, chocolate creams; sauces including fruit flavored sauces, chocolate sauces or mixtures thereof; edible gels; cremes including butter cremes, flour pastes, whipped cream or mixtures thereof; jams including strawberry jam, marmalade or mixtures thereof; breads including sweet breads or other starch products or mixtures thereof; spice; general condiments including seasoned soy sauce used on roasted meats, roast fowl, barbecued meat or mixtures thereof, as well as tomato catsup, sauces, noodle broth or mixtures thereof; processed agricultural products, livestock products or seafood; processed meat products which are sausage or mixtures thereof; retort food products, pickles, preserves boiled in soy sauce, delicacies, side dishes; snacks which are potato chips, cookies or mixtures thereof; wafer, waffle, cornets, bars, wafer sheet; cereal products, granola; baby food;
- said pharmaceutical products are selected from the group comprising drugs or quasi-drugs that are administered orally or used in the oral cavity, wherein the drug may be in solid, liquid, gel or gas form which is a pill, tablet, spray, capsule, syrup, drop, troche agent, sachet, powder or mixtures thereof; herbal products, vitamins; nutraceutical products comprising any food or part of a food that may provide medicinal or health benefits, including the prevention and treatment of disease;
- said oral products are selected from the group comprising hygienic or cosmetic products; personal care products which are oral compositions used in the oral cavity, mouth freshening agents, gargling agents, mouth rinsing agents, toothpaste, tooth polish, dentrifices, mouth sprays, teeth-whitening agents or mixtures thereof;
- said dietetic products are selected from the group comprising dietary supplement;
- said veterinary products are selected from the group comprising animal feed.

## Patentansprüche

1. Natürliche Süßstoffzusammensetzung, umfassend:
a) Apfelkonzentrat einer mittleren Teilchengröße (d50) von mehr als 100 µm,
b) Luo Han Guo-Konzentrat einer mittleren Teilchengröße (d50) von mehr als 1 µm.

2. Natürliche Süßstoffzusammensetzung nach Anspruch 1, umfassend:
a) Apfelkonzentrat einer mittleren Teilchengröße (d50) zwischen 100 µm und 500 µm,
b) Luo Han Guo-Konzentrat einer mittleren Teilchengröße (d50) zwischen 1 µm und 50 µm.

3. Natürliche Süßstoffzusammensetzung nach Anspruch 1, umfassend:
c) Apfelkonzentrat eines Teilchengrößenverhältnisses von (d50) bis (d90), das zwischen 0,0 und 0,80 beträgt,
d) Luo Han Guo-Konzentrat eines Teilchengrößenverhältnisses von (d50) bis (d90), das zwischen 0,20 und 0,70 beträgt.

4. Natürliche Süßstoffzusammensetzung nach Anspruch 1, umfassend Apfelkonzentrat und Luo Han Guo-Konzentrat, bei der das Teilchengrößenverhältnis (d50) von Luo Han Guo-Konzentrat zu Apfelkonzentrat zwischen 0,01 und 0,1 beträgt.

5. Natürliche Süßstoffzusammensetzung nach Anspruch 1, des Weiteren umfassend Inulin, Erythrit, Kokoszucker, Laktose, Johannisbrotkernmehl; Rebaudiosid A, Rebaudiosid B, Rebaudiosid C, Rebaudiosid D, Rebaudiosid E, Rebaudiosid F, Dulcosid A, Dulcosid B, Rubusosid, Isomogrosid V, 11-Oxomogrosid, Steviolglukoside, Stevia, Steviosid, Mogrosid IV, Mogrosid V, Mogrosid VI, Siamenosid, Monatin und dessen Salze (Monatin SS, RR, RS, SR), Curculin, Glycyrrhizin, Glycyrrhizinsäure und deren Salze, Thaumatin, Monellin, Mabinlin, Brazzein, Hernandulein, Phyllodulcin, Glycyphyllin, Phloridzin, Trilobatin, Baiyunosid, Osladin, Polypodosid A, Pterocaryosid A, Pterocaryosid B, Mukuroziosid, Phlomisosid I, Periandrin I, Abrusosid A, Cyclocariosid I; einen Süßstoff aus der Gruppe umfassend Stevia rebaudiana, Glycyrrhiza glabra, Siraitia grosvenorii; Saccharose, flüssige Saccharose, Glukose, flüssige Glukose, Fruktose, flüssige Fruktose, Dextrose, Galaktose, Laktulose, Laktose, Cellobiose, Kojibiose, Nigeriose, Isomaltose, β,β-Trehalose, α,β-Trehalose, Tagatose, Sophorose, Laminaribiose, Gentiobiose, Turanose, Maltose, Maltutose, Palatinose, Gentiobiulose, Mannobiose, Melibiose, Melibiulose, Rhamnose, Ribose, Rutinose, Rutinulose, Trehalose, Xylobiose, Xylose, Maissirupe, Fructooligosaccharide; Erythrit, Glykol, Glycerol, Threitol, Arabitol, Xylitol, Ribitol, Mannitol, Sorbitol, Dulcitol, Iditol, Isomalt, Maltitol, Lactitol, Polyglycitol; Apfelschalenextrakt (Apfelphenon) oder Mischungen davon.

6. Natürliche Süßstoffzusammensetzung nach Anspruch 5, wobei die Zusammensetzung Apfelkonzentrat, Luo Han Guo-Konzentrat und Inulin umfasst.

7. Natürliche Süßstoffzusammensetzung nach Anspruch 5, wobei die Zusammensetzung Apfelkonzentrat, Luo Han Guo-Konzentrat und Erythrit umfasst.

8. Natürliche Süßstoffzusammensetzung nach Anspruch 5, wobei die Zusammensetzung Apfelkonzentrat, Luo Han Guo-Konzentrat und Kokoszucker umfasst.

9. Natürliche Süßstoffzusammensetzung nach Anspruch 5, wobei die Zusammensetzung Apfelkonzentrat, Luo Han Guo-Konzentrat und Laktose umfasst.

10. Natürliche Süßstoffzusammensetzung nach Anspruch 5, wobei die Zusammensetzung Apfelkonzentrat, Luo Han Guo-Konzentrat und Johannisbrotkernmehl umfasst.

11. Natürliche Süßstoffzusammensetzung nach Anspruch 5, wobei die Zusammensetzung Apfelkonzentrat, Luo Han Guo-Konzentrat, Inulin und Erythrit umfasst.

12. Natürliche Süßstoffzusammensetzung nach Anspruch 5, wobei die Zusammensetzung Apfelkonzentrat, Luo Han Guo-Konzentrat, Inulin, Erythrit und Kokoszucker umfasst.

13. Natürliche Süßstoffzusammensetzung nach Anspruch 5, wobei die Zusammensetzung Apfelkonzentrat, Luo Han Guo-Konzentrat, Inulin, Erythrit und Johannisbrotkernmehl umfasst.

14. Natürliche Süßstoffzusammensetzung nach Anspruch 5, wobei die Zusammensetzung Apfelkonzentrat, Luo Han Guo-Konzentrat, Inulin, Erythrit, Kokoszucker und Laktose umfasst.

15. Natürliche Süßstoffzusammensetzung nach Anspruch 5, wobei die Zusammensetzung Apfelkonzentrat, Luo Han Guo-Konzentrat, Inulin, Erythrit, Kokoszucker, Laktose und Johannisbrotkernmehl umfasst.

16. Verwendung der natürlichen Süßstoffzusammensetzung umfassend Apfelkonzentrat und Luo Han Guo-Konzentrat nach einem der Ansprüche 1-15 in Getränke-, Lebensmittel-, Arzneimittel-, Mundpflege-, Diät- oder veterinärmedizinischen Produkten.

17. Verwendung der natürlichen Süßstoffzusammensetzung nach Anspruch 16, wobei besagte Produkte in fester, flüssiger oder halbfester Form vorliegen.

18. Verwendung der natürlichen Süßstoffzusammensetzung nach Anspruch 17, wobei:
- besagte Getränke ausgewählt sind aus der Gruppe umfassend Cola-Getränke, Ginger Ales, Rootbeers, Apfelweine, nichtalkoholische Getränke mit Fruchtgeschmack, nichtalkoholische Getränkepulver oder Mischungen davon; Fruchtsäfte auf Frucht- oder Gemüsebasis, Fruchtsäfte einschließlich gepresste Säfte oder Mischungen davon, Fruchtsäfte mit Fruchtteilchen, Fruchtgetränke, Fruchtsaftgetränke, fruchtsafthaltige Getränke, Getränke mit Fruchtgeschmacksmitteln, Gemüsesäfte, gemüsehaltige Säfte, und Mischsäfte aus Früchten und Gemüsen; Getränke, Sportdrinks, Energydrinks, Near-Water-Getränke oder Mischungen davon; teeartige oder bevorzugte Getränke wie Kaffee, Kakao, Schokolade, schwarzer Tee, grüner Tee, Oolong-Tee oder Mischungen davon; Getränke mit Milchbestandteilen, wie Milchgetränke, Kaffee mit Milchbestandteilen, Milchkaffee, Tee mit Milch, fruchtige Milchgetränke, Trinkjoghurt, Getränke mit Milchsäurebakterien oder Mischungen davon; Milchprodukte; alkoholische Getränke wie Wein, Bier, Apfelwein; destillierte Getränke wie Spirituosen, Schnaps;
- besagte Lebensmittelprodukte ausgewählt sind aus der Gruppe umfassend Backwaren; Desserts wie Joghurt, Wackelpuddinge, Trinkgelees, Puddinge, Bayrische Creme, Blancmange, Kuchen, Brownies, Mousse oder Mischungen davon; gesüßte Lebensmittelprodukte, die am Nachmittag oder nach Hauptmahlzeiten gegessen werden; gefrorene Lebensmittel; kalte Süßwaren, Arten von Eiscreme wie Speiseeis, Milcheis, Lacto-Eis oder Mischungen davon; Eiszubereitungen wie Sorbets, Eisdesserts oder Mischungen davon; Eiscreme; allgemeine Süßwaren, gebackene Konfektwaren oder gedämpfte Konfektwaren wie Kuchen, Cracker, Kekse, mit süßer Bohnenpaste gefüllte Teigkugeln oder Mischungen davon; Reiskuchen und Snacks; Nuß- und Erdnußbutter; Tischgewürze; allgemeines Zuckerkonfekt wie Kaugummi, Hartkaramellen, Kaubonbons, Minzbonbons, Nougatkonfekt, Geleebohnen oder Mischungen davon; Schokoladen, Schokoladencremes; Soßen einschließlich Fruchtsaucen, Schokoladensaucen oder Mischungen davon; Speisegelarte; Cremes einschließlich Buttercremes, Mehlpasten, Schlagsahne oder Mischungen davon; Konfitüren einschließlich Erdbeerkonfitüre, Orangenmarmelade oder Mischungen davon; Brote einschließlich Süßbrote oder andere Stärkeprodukte oder Mischungen davon; Würze; allgemeine Würzmittel einschließlich gewürzter Sojasauce zur Verwendung auf gebratenem Fleisch, gebratenem Geflügel, gegrilltem Fleisch oder Mischungen davon, sowie Tomatenketchup, Soßen, Nudelbrühe oder Mischungen davon; verarbeitete landwirtschaftliche Erzeugnisse, tierische Erzeugnisse oder Meeresfrüchte; verarbeitete Fleischerzeugnisse wie Wurst oder Mischungen davon; Retortenlebensmittel, Pickles, in Sojasauce gekochte Konserven, Delikatessen, Beilagen; Snacks wie Kartoffelchips, Kekse oder Mischungen davon; Oblaten, Waffeln, Spitztüten, Riegel, Waffelblätter; Getreideprodukte, Müsli, Babynahrung;
- besagte Arzneimittelprodukte ausgewählt sind aus der Gruppe umfassend Arzneimittel oder Quasi-Arzneimittel, die oral verabreicht oder in der Mundhöhle verwendet werden, wobei das Arzneimittel in fester, flüssiger, gelartiger oder gasartiger Form vorliegen kann, wie eine Pille, eine Tablette, ein Spray, eine Kapsel, ein Sirup, Tropfen, Tropfmittel, Beutel, Pulver oder Mischungen davon; pflanzliche Produkte, Vitamine; nutrazeutische Produkte, die jedwede Lebensmittel oder Teile eines Lebensmittels umfassen, die von medizinischem oder gesundheitlichem Nutzen sein können, einschließlich der Prävention und Behandlung von Krankheiten;
- besagte Mundpflegeprodukte ausgewählt sind aus der Gruppe umfassend Hygiene- oder Kosmetikprodukte; Körperpflegeprodukte, bei denen es sich um Mundpflegepräparate für den Mundraum handelt, Munderfrischungsmittel, Gurgelmittel, Mundspülmittel, Zahnpasta, Zahnpolierpaste, Zahnputzmittel, Mundsprays, Zahnbleichmittel oder Mischungen davon;
- besagte Diätprodukte ausgewählt sind aus der Gruppe umfassend Nahrungsergänzungsmittel;
- besagte veterinärmedizinische Produkte ausgewählt sind aus der Gruppe der Tierfuttermittel.

## Revendications

1. Composition édulcorante naturelle comprenant :
a) du concentré de pomme ayant une taille de particule médiane (d50) supérieure à 100 µm,
b) du concentré de Luo Han Guo ayant une taille de particule médiane (d50) supérieure à 1 µm.

2. Composition édulcorante naturelle selon la revendication 1, comprenant :
a) du concentré de pomme ayant une taille de particule médiane (d50) entre 100 et 500 µm,
b) du concentré de Luo Han Guo ayant une taille de particule médiane (d50) entre 1 et 50 µm.

3. Composition édulcorante naturelle selon la revendication 1, comprenant :
a) du concentré de pomme ayant un rapport de taille de particule de (d50) à (d90) qui est entre 0,30 et 0,80,
b) du concentré de Luo Han Guo ayant un rapport de taille de particule de (d50) à (d90) qui est entre 0,20 et 0,70.

4. Composition édulcorante naturelle selon la revendication 1, comprenant du concentré de pomme et du concentré de Luo Han Guo, le rapport de taille de particule de d(50) du concentré de Luo Han Guo au concentré de pomme étant entre 0,01 et 0,1.

5. Composition édulcorante naturelle selon la revendication 1, comprenant en outre de l'inuline, de l'érythritol, du sucre de noix de coco, du lactose, de la gomme de caroube ; du rébaudioside A, du rébaudioside B, du rébaudioside C, du rébaudioside D, du rébaudioside E, du rébaudioside F, du dulcoside A, du dulcoside B, du rubusoside, de l'isomogroside-V, du 11-oxomogroside, des glycosides de stéviol, du stévia, du stévioside, du mogroside IV, du mogroside V, du mogroside VI, du siaménoside, de la monatine et ses sels (monatine SS, RR, RS, SR), de la curculine, de la glycyrrhizine, de l'acide glycyrrhizique et ses sels, de la thaumatine, de la monelline, de la mabinline, de la brazzéine, de l'hernanduléine, de la phyllodulcine, de la glycyphylline, de la phloridzine, de la trilobatine, du baiyunoside, de l'osladine, du polypodoside A, du ptérocaryoside A, du ptérocaryoside B, du mukurozioside, du phlomisoside I, de la périandrine I, de l'abrusoside A, du cyclocarioside I ; un agent édulcorant qui est extrait du groupe comprenant Stevia rebaudiana, Glycyrrhiza glabra, Siraitia grosvenorii ; du saccharose, du saccharose liquide, du glucose, du glucose liquide, du fructose, du fructose liquide, du dextrose, du galactose, du lactulose, du lactose, du cellobiose, du kojibiose, du nigérose, de l'isomaltose, du .bêta.,.bêta.-tréhalose, de l'.alpha.,.bêta.-tréhalose, du tagatose, du sophorose, du laminaribiose, du gentiobiose, du turanose, du maltose, du maltulose, du palatinose, du gentiobiulose, du mannobiose, du mélibiose, du mélibiulose, du rhamnose, du ribose, du rutinose, du rutinulose, du tréhalose, du xylobiose, du xylose, des sirops de maïs, des fructo-oligosaccharides ; de l'érythritol, du glycol, du glycérol, du thréitol, de l'arabitol, du xylitol, du ribitol, du mannitol, du sorbitol, du dulcitol, de l'iditol, de l'isomalt, du maltitol, du lactitol, du polyglycitol ; de l'extrait de peau de pomme (« Applephenon ») ou des mélanges de ceux-ci.

6. Composition édulcorante naturelle selon la revendication 5, la composition comprenant du concentré de pomme, du concentré de Luo Han Guo et de l'inuline.

7. Composition édulcorante naturelle selon la revendication 5, la composition comprenant du concentré de pomme, du concentré de Luo Han Guo et de l'érythritol.

8. Composition édulcorante naturelle selon la revendication 5, la composition comprenant du concentré de pomme, du concentré de Luo Han Guo et du sucre de noix de coco.

9. Composition édulcorante naturelle selon la revendication 5, la composition comprenant du concentré de pomme, du concentré de Luo Han Guo et du lactose.

10. Composition édulcorante naturelle selon la revendication 5, la composition comprenant du concentré de pomme, du concentré de Luo Han Guo et de la gomme de caroube.

11. Composition édulcorante naturelle selon la revendication 5, la composition comprenant du concentré de pomme, du concentré de Luo Han Guo, de l'inuline et de l'érythritol.

12. Composition édulcorante naturelle selon la revendication 5, la composition comprenant du concentré de pomme, du concentré de Luo Han Guo, de l'inuline, de l'érythritol et du sucre de noix de coco.

13. Composition édulcorante naturelle selon la revendication 5, la composition comprenant du concentré de pomme, du concentré de Luo Han Guo, de l'inuline, de l'érythritol et de la gomme de caroube.

14. Composition édulcorante naturelle selon la revendication 5, la composition comprenant du concentré de pomme, du concentré de Luo Han Guo, de l'inuline, de l'érythritol, du sucre de noix de coco et du lactose.

15. Composition édulcorante naturelle selon la revendication 5, la composition comprenant du concentré de pomme, du concentré de Luo Han Guo, de l'inuline, de l'érythritol, du sucre de noix de coco, du lactose et de la gomme de caroube.

16. Utilisation de la composition édulcorante naturelle comprenant du concentré de pomme et du concentré de Luo Han Guo selon l'une quelconque des revendications 1 à 15 dans des produits à boire, alimentaires, pharmaceutiques, sous forme orale, diététiques ou vétérinaires.

17. Utilisation de la composition édulcorante naturelle selon la revendication 16, dans laquelle lesdits produits sont solides, liquides ou semi-solides.

18. Utilisation de la composition édulcorante naturelle selon la revendication 17, dans laquelle :
- lesdits produits à boire sont choisis dans le groupe comprenant les colas, les bières au gingembre, les racinettes, les cidres, les boissons non alcoolisées aromatisées aux fruits, les boissons non alcoolisées en poudre ou les mélanges de ceux-ci ; les jus de fruits provenant de fruits ou de légumes, les jus de fruits comprenant des jus de pression ou des mélanges de ceux-ci, les jus de fruits contenant des particules de fruits, les boissons aux fruits, les boissons à base de jus de fruits, les boissons contenant des jus de fruits, les boissons avec des aromatisants aux fruits, les jus de légumes, les jus contenant des légumes, et les jus mélangés contenant des fruits et des légumes ; les boissons, les boissons pour le sport, les boissons énergisantes, les quasi-eaux (« near water ») ou les mélanges de celles-ci ; les boissons de type thé ou de type boisson favorite qui sont le café, le cacao, le chocolat, le thé noir, le thé vert, le thé Oolong ou les mélanges de ceux-ci ; les boissons contenant des composants du lait qui sont les boissons lactées, le café contenant des composants du lait, le café au lait, le thé au lait, les boissons lactées aux fruits, le yaourt à boire, les boissons à bactéries lactiques ou les mélanges de ceux-ci ; les produits laitiers ; les boissons alcoolisées qui sont le vin, la bière, le cidre ; les boissons distillées qui sont les spiritueux, les boissons fortement alcoolisées ;
- lesdits produits alimentaires sont choisis dans le groupe comprenant les produits de boulangerie-pâtisserie ; les desserts qui sont le yaourt, les gelées, les gelées à boire, les puddings, la crème bavaroise, le blanc-manger, les gâteaux, les brownies, la mousse ou les mélanges de ceux-ci ; les produits alimentaires sucrés pris à l'heure du thé ou après les repas ; les aliments congelés ; les confiseries froides, les types de glace qui sont la glace, la glace au lait, la lacto-glace ou les mélanges de celles-ci ; les confiseries glacées qui sont les sorbets, les glaces pour le dessert ou les mélanges de ceux-ci ; la crème glacée ; les confiseries en général, les confiseries cuites au four ou confiseries cuites à la vapeur qui sont les gâteaux, les biscuits salés, les biscuits, les petits pains fourrés de confiture de fèves ou les mélanges de ceux-ci ; les galettes de riz et les en-cas ; le beurre de noix et de cacahuète ; les produits de table ; les confiseries sucrées en général qui sont la gomme à mâcher, les bonbons durs, les bonbons mous, les menthes, le nougat, les dragées tendres ou les mélanges de ceux-ci ; les chocolats, les crèmes au chocolat ; les sauces comprenant les sauces aromatisées aux fruits, les sauces au chocolat ou les mélanges de celles-ci ; les gels comestibles ; les crèmes comprenant les crèmes au beurre, les pâtes à base de farine, la crème fouettée ou les mélanges de celles-ci ; les confitures comprenant la confiture de fraise, la marmelade ou les mélanges de celles-ci ; les pains comprenant les pains sucrés ou autres produits à base d'amidon ou les mélanges de ceux-ci ; les épices ; les condiments en général comprenant la sauce de soja assaisonnée utilisée sur les viandes rôties, la volaille rôtie, la viande cuite au barbecue ou les mélanges de celles-ci, ainsi que le ketchup de tomate, les sauces, le bouillon de nouilles ou les mélanges de ceux-ci ; les produits agricoles traités, les produits de l'élevage ou les fruits de mer; les produits à base de viande transformée qui sont les saucisses ou les mélanges de celles-ci ; les produits alimentaires stérilisés en autoclave, les cornichons, les conserves cuites dans de la sauce de soja, les mets délicats, les accompagnements ; les en-cas qui sont les chips de pomme de terre, les cookies ou les mélanges de ceux-ci ; les gaufrettes, les gaufres, les cornets, les barres, les feuilles de gaufrettes ; les produits à base de céréales, le muesli ; les aliments pour bébés ;
- lesdits produits pharmaceutiques sont choisis dans le groupe comprenant les médicaments ou quasi-médicaments qui sont administrés par voie orale ou utilisés dans la cavité buccale, le médicament pouvant être sous forme solide, liquide, de gel ou gazeuse, qui est une pilule, un comprimé, une pulvérisation, une capsule, un sirop, une goutte, une tablette, un sachet, une poudre ou les mélanges de ceux-ci ; les produits à base d'herbes médicinales, les vitamines ; les produits nutraceutiques comprenant tout aliment ou partie d'un aliment qui peuvent offrir des bienfaits médicinaux ou pour la santé, comprenant la prévention et le traitement d'une maladie ;
- lesdits produits sous forme orale sont choisis dans le groupe comprenant les produits hygiéniques ou cosmétiques ; les produits de soins personnels qui sont les compositions orales utilisées dans la cavité buccale, les agents de rafraîchissement de la bouche, les agents pour gargarisme, les agents de rinçage de la bouche, la pâte dentifrice, les agents pour polissage des dents, les dentifrices, les pulvérisations buccales, les agents de blanchiment des dents ou les mélanges de ceux-ci ;
- lesdits produits diététiques sont choisis dans le groupe comprenant les suppléments diététiques ;
- lesdits produits vétérinaires sont choisis dans le groupe comprenant les aliments pour animaux.
